# EUROPÄISCHE PATENTSCHRIFT

(11) **EP 0 951 904 B1**
(45) Veröffentlichungstag und Bekanntmachung des Hinweises auf die Patenterteilung: **09.08.2006**
(21) Anmeldenummer: 99250129.6
(22) Anmeldetag: 22.04.1999
(51) Int. Cl.: A61K 31/00, A61K 31/045, A61K 31/20, A61Q 19/00

(54) **Mittel zur Verbesserung der Mikrozirkulation der Haut**
Agent for enhancing dermal microcirculation
Médicament et pour augmenter la microcirculation cutanéene

(30) Priorität: 23.04.1998 DE 19818914
(43) Veröffentlichungstag der Anmeldung: 27.10.1999
(73) Patentinhaber: Gabriele Wyeth KG Kosmetik Berlin, 13409 Berlin (DE)
(72) Erfinder: Hörner, Wolf-Dieter,, DE-13465 Berlin (DE); Gäbelein, Klaus, Dr.rer.nat.,, 82284 Grafrath (DE)
(74) Vertreter: Sternagel, Fleischer, Godemeyer & Partner

(56) Entgegenhaltungen:
- WO-A-96/25409
- FR-A- 2 687 312
- FR-A- 2 750 331

## Beschreibung

Die Erfindung bezieht sich auf ein Mittel zur Verbesserung der Mikrozirkulation der Haut, das als Wirkstoff mindestens eine kosmetisch und pharmazuetisch verträgliche, aktivierten Sauerstoff enthaltende organische Verbindung enthält.

Unter aktivierten Sauerstoff enthaltenden Verbindungen werden Verbindungen verstanden, in die eine Peroxidgruppe eingebracht worden ist, vorzugsweise mit Hilfe von Ozon.

Es ist z.B. bekannt, daß bei der Ozonierung von olefinisch ungesättigten organischen Verbindungen sogenannte Ozonide gebildet werden, die jedoch im allgemeinen wenig stabil sind. Erfolgt die Ozonierung jedoch in Gegenwart eines hydroxylgruppenhaltigen Lösungsmittels, werden stabile Verbindungen, die Acetalperoxide, erhalten (DE-B 10 75 801, DE-B 11 66 975, DE-A 16 68 144 und DE-A 41 13 389).

Weiter ist bekannt, Peroxy-aldehyde und Peroxy-carbonsäuren durch Oxidation von gesättigten Alkoholen, Aldehyden oder Ketonen mit Ozongas oder ozonhaltigen Gasen herzustellen (DE-A 36 43 323).

Für diese Verbindungen ist eine hohe bakterizide Wirkung beschrieben, die mit einer ausgezeichneten Hautverträglichkeit einhergeht. Es wird daher vorgeschlagen, sie als Desinfektionsmittel in Kosmetika und pharmazeutischen Erzeugnissen, zur Wundbehandlung, z.B. zur Behandlung von antibiotika-resistenten eitrigen Fisteln, sowie als Sauerstofflieferant für die Haut einzusetzen (DE-B 10 75 801, DE-A 16 68 144, K. Gäbelein, Seifen, Öle, Wachse, 112.Jg.,Seite 17, 1986) .

Ein Einfluß von peroxidieren Ölen, Benzolproxyde sowie von Ozoniden auf die Mikrozirkulation der Haut ist in dem Druckschriften FR-A-2 750 331, FR-A-2 687 312 bzw. WO 96/25409A erwähnt. Die Druckshrift WO 96/25409 A beschreibt, dass derartige Ozonide durch Ozonierung einer ungesätigten Fettsäure vorbereitet werden.

Unter Mikrozirkulation wird die im Bereich der Endstrombahn, der aus Arteriolen, Kapillaren und postkapillaren Venen (Venolen) bestehende Abschnitt des Gefäßsystems, subkutan verlaufende Blutzirkulation verstanden.

Über diese erfolgt die nutritive Blutversorgung durch Stoffaustausch zwischen Blut und Gewebe sowie die Aufrechterhaltung des thermalen und des Ionenmilieus. Störungen der Mikrozirkulation haben daher gravierende Auswirkungen auf die Versorgung des Gewebes mit essentiellen Stoffen.

Beispielsweise nimmt bei verlangsamter Strömung die Suspensionsstabilität des Bluts ab, was Plasma-"skimming" zur Folge hat, ein Phänomen, bei dem es an den Verzweigungen der Kapillaren zur teilweisen oder vollständigen Trennung von Plasma und zellulären Blutbestandteilen kommt, so daß einzelne Kapillaren nur mit Plasma durchströmt werden, wodurch die Versorgung in diesen Bereichen beeinträchtigt wird.

Die Mikrozirkulation der Haut übt somit auf die Hautfunktion einen maßgeblichen Einfluß aus und steht in engem Zusammenhang mit der Alterung der Haut.

Es ist Aufgabe der vorliegenden Erfindung, ein Mittel zur Verfügung zu stellen, das die Mikrozirkulation der Haut verbessern kann und infolge davon die Hautfunktionen positiv zu stimulieren vermag.

Die technische Aufgabe wird gelöst durch die Verwendung einer kosmetisch und dermatologisch verträglichen organischen Verbindung mit aktiviertem Sauerstoff zur Herstellung eines Präparates zur Verbesserung der Mikrozirkulation der Haut, wobei die Verbindungen mit aktiviertem Sauerstoff Acetalperoxide sind, die durch Ozonierung einer gesättigten oder ungesättigten Fettsäure oder eines Esters davon in Gegenwart eines Alkohols oder eines teilveretherten oder teilveresterten Polyols erhalten werden.

Vollkommen überraschend wurde nun gefunden, daß aktivierten Sauerstoff enthaltende organische Verbindungen die Mikrozirkulation der Haut steigern können und damit den ungestörten Blutfluß und die Versorgung der Haut mit wichtigen Nährstoffen unterstützen. Besonders positive Effekte konnten für die strapazierte und alternde Haut nachgewiesen werden.

Die vorliegende Erfindung betrifft somit ein Mittel zur Verbesserung der Mikrozirkulation der Haut, das als Wirkstoff mindestens eine kosmetisch und dermatologisch verträgliche, aktivierten Sauerstoff enthaltende organische Verbindung enthält.

In einer bevorzugten Ausführungsform ist das Acetalperoxid durch Ozonierung von Sorbinsäure in Ethyldiglykol erhältlich ist. Vorzugsweise ist das Acetalperoxid in Ethyldiglykol gelöst.

Weiterhin wird bevorzugt, dass das Acetalperoxid in dem Mittel in einer Menge von 0,5 bis 20 Gew.-%, bezogen auf das Gesamtgewicht des Mittels enthalten ist.

Die Aufgabe wird zudem gelöst durch eine pharmazeutische Zusammensetzung zur Verbesserung der Mikrozirkulation der Haut enthaltend Acetalperoxide und eine Fettsäure und/oder einen Fettsäureester.

Nachfolgend werden derartige Verbindungen auch Verbindungen mit aktiviertem Sauerstoff genannt.

Dieses Mittel eignet sich insbesondere zur Förderung der Versorgung von strapazierter und alternder Haut. In bezug auf jugendliche bzw. junge Haut ist die Unterstützung des ungestörten Blutflusses hervorzuheben, wodurch möglichen Störungen vorgebeugt werden kann.

Das Mittel kann in einem weiten Anwendungsbereich erfolgreich eingesetzt werden, wie z.B. der Kosmetik, Pharmazie, insbesondere Dermatologie, und auch der kosmetischen und medizinischen Behandlung von Tieren.

Erfindungsgemäß handelt es sich bei den aktivierten Sauerstoff enthaltenden organischen Verbindungen um Verbindungen mit einer Peroxidgruppe, die kosmetisch und dermatologisch verträglich sind.

Beispiele hierfür sind die Acetalperoxide. Besonders geeignete Vertreter sowie Verfahren zur deren Herstellung sind in DE-B 10 75 801, DE-B 11 66 975, DE-A 16 68 144 und DE-A 41 13 389 beschrieben.

Die Acetalperoxide werden durch Ozonierung von olefinisch ungesättigten organischen Verbindungen in Gegenwart von Alkoholen oder vorzugsweise teilveretherten oder teilveresterten Polyolen, wie Glykolen, Polyglykolen und Glycerin erhalten. In diesem Fall beteiligen sich der Alkohol bzw. das Polyol an der Reaktion und es kommt zur Ausbildung von Halbacetalen 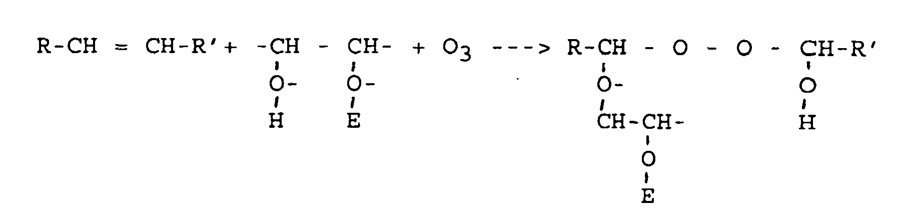 wobei R und R', unabhängig voneinander, gleich oder verschieden, einen organischen Rest bedeuten, der als C1-C30-Kohlenwasserstoff, -Alkohol, -Aldehyd, -Keton, -Säure oder deren Ester vorliegen kann, und E ein Rest eines organischen Alkohols oder einer Säure darstellt, bzw. C1-C10, vorzugsweise C1-C4, Alkyl oder R"-CO-darstellt, wobei R" vorzugsweise C1-C10, beispielsweise C1-C4-Alkyl ist.

Bevorzugte Beispiele für die ungesättigte organische Verbindung sind ungesättigte Fettsäuren und deren Ester wie Sorbinsäure, Ölsäure, Oleyloleat, Leinölfettsäureethylester, Rizinusöl, Olivenöl, Sojaöl etc. sowie Terpineol, wobei Sorbinsäure besonders bevorzugt ist.

Geeignete Alkohole sind beispielsweise niedrige Alkohole, insbesondere C1-C6-Monoole, Methanol, Ethanol und Propanol.

Besonders vorteilhaft können zur Reaktion mit der olefinisch ungesättigten Verbindung teilveresterte oder veretherte Polyole, wie Glykole und Polyglykole, z.B. Ethylen-, Propylen- und Butylenglykol, sowie Glycerin verwendet werden. Beispiele hierfür sind Ethyl-, Propyl- und Butylglykolether, Ethyldiglykol, 1,2-Propylenglykol-mono-etylether und Glycerindiacetat.

Erfindungsgemäß besonders bevorzugt ist ein Acetalperoxid, das aus Sorbinsäure in Ethyldiglykol erhalten wird.

Weiter können Peroxy-aldehyde und Peroxy-carbonsäuren, wie sie z.B. in der DE-A 36 43 323 beschrieben sind, verwendet werden.

Es können auch Kombinationen von verschiedenen Verbindungen mit aktiviertem Sauerstoff eingesetzt werden.

Im Gegensatz zu den hautaggressiven und toxischen Industrieperoxiden, wie Wasserstoffperoxid, sind die erfindungsgemäß verwendeten Verbindungen ausgesprochen hautverträglich und nicht toxisch.

Diese außergewöhnliche Hautverträglichkeit wird darauf zurückgeführt, daß es sich bei den erfindungsgemäß verwendeten Verbindungen mit aktiviertem Sauerstoff um Verbindungen handelt, wie sie ähnlich in der Natur selbst gebildet werden, wenn sich atmosphärisches Ozon in Gegenwart von hydroxylgruppenhaltigen Substanzen an pflanzliche Materialen bindet.

Die erfindungsgemäßen Mittel zur Verbesserung der Mikrozirkulation der Haut können neben der aktivierten Sauerstoff enthaltenden Verbindung selbstverständlich weitere, in solchen Mitteln übliche Träger-, Wirk- und Zusatzstoffe enthalten, die sich je nach der gewünschten Anwendung des Mittels bzw. der Verbindung mit aktiviertem Sauerstoff bestimmen.

Die Auswahl dieser Zusätze sowie die Verfahren zur Formulierung erfolgen nach den üblichen, dem Galeniker geläufigen Methoden.

Das Mittel kann in beliebiger Applikationsform eingesetzt werden, z.B. in wäßriger oder alkoholischer Lösung, Mischformen davon, in fester oder nicht-fester Form, Öl-in-Wasser oder Wasser-in-Öl Emulsion und Mischformen davon, als Mikroemulsionen, Gele etc.. Es kann beispielsweise als Creme, Öl, Spray, Schaumaerosol oder als Badezusatz eingesetzt werden.

Der Anteil an aktivierten Sauerstoff enthaltender Verbindung in dem Mittel variiert dabei je nach Verwendungszweck sowie Applikationsform.

Im allgemeinen hat es sich als ausreichend erwiesen, die Verbindung dem Mittel in einem Anteil von 0,5 bis 20 Gew.-%, bezogen auf das Gesamtgewicht der Mittels, zuzusetzen.

Vorzugsweise liegt der Anteil bei 1 bis 12 Gew.-%, besonders bevorzugt bei 2 bis 8 Gew.-% und insbesondere bei 3 bis 6 Gew.-%.

Wird das Mittel bei der Anwendung verdünnt, wie z.B. im Fall eines Badezusatzes, so kann der Anteil an der Verbindung mit aktiviertem Sauerstoff bis 20 Gew.-% betragen. Bei Bedarf kann der Anteil selbstverständlich auch höher sein.

In einer weiteren Ausführungsform enthält das Mittel neben der Verbindung mit aktiviertem Sauerstoff zusätzlich gesättigte und/oder ungesättigte Fettsäuren und/oder deren Ester.

Beispiele hierfür sind die Fettsäuren bzw. Fettsäureester, die vorstehend zur Herstellung der Verbindung mit aktiviertem Sauerstoff beschrieben sind.

Der Anteil an Fettsäure und/oder Fettsäureester kann dabei in der gleichen Größenordnung wie vorstehend für die Verbindung mit aktiviertem Sauerstoff beschrieben, liegen.

Durch den kombinierten Einsatz der Verbindung mit aktiviertem Sauerstoff und der Fettsäure/ester kann der positive Effekt auf die Haut und zudem die Stabilität der Fettsäure/ester erhöht werden.

Die erfindungsgemäß verwendeten Acetalperoxide können auch vorteilhafterweise in Lösung in einem Alkohol, Polyol, teilveresterten bzw. teilveretherten Polyolen, wie sie z.B. vorstehend für die Herstellung der Acetalperoxide beschrieben worden sind, als solche oder mit weiteren Zusätzen eingesetzt werden.

Beispielsweise wird das erfindungsgemäß bevorzugte Acetalperoxid der Sorbinsäure mit Ethylendiglykol in einer erfindungsgemäß besonders bevorzugten Ausführungsform in seiner 4%-igen Lösung in Ethyldiglycol eingesetzt (Ozonid SV^{R}).

Nachstehend wird die vorliegende Erfindung am Beispiel der Auswirkung des erfindungsgemäßen Mittels auf die Mikrozirkulation der Haut veranschaulicht.

Die Testpersonen waren Frauen im Alter von 46 bis 55 Jahren mit normaler und gesunder Haut (sog.Mitteltyp). Es wurden auf dem rechten und linken Schulterbereich des Rückens je zwei untereinanderliegende Test- und Kontrollarealpaare ausgewählt, die Testsubstanz wurde auf die Testareale aufgebracht und die Änderung der Mikrozirkulation wurde im Vergleich zu den Kontrollarealen bestimmt, auf die keine Testsubstanz aufgebracht worden waren.

Die Applikation der Testsubstanzen erfolgte unter Berücksichtigung der DIN 67 501 (Dezember 1985): Experimentelle dermatologische Bewertung des Erythmenschutzes von externen Sonnenschutzmitteln für die menschliche Haut".

Die Auswertung erfolgte jeweils vor und nach Applikation der Testsubstanz, wobei eine intravitalmikroskopische Untersuchungseinheit im kombinierten Auflicht- Durchlicht-Verfahren mit selektiven Lichtgeneratoren und selektiver Filterung verwendet wurde.

Als Testsubstanz wurde eine Pflegecreme mit Ozonid SV^{R} verwendet.

Im Ergebnis zeigte sich bei Anwendung der Pflegecreme Ozonid SV^{R} eine Erhöhung der mittleren Strömungsgeschwindigkeit der roten Blutzellen in den kapillaren Strömen und eine Verbesserung der Verteilung des Bluts in der Mikrozirkulation der Haut, wodurch der Funktionszustand der Mikrozirkulation in der Haut positiv beeinflußt wurde.

Die Erfindung lehrt im einzelnen Gegenstände gemäß den Patentansprüchen 1 bis 5. Die vorstehenden Ausführungen zum Mittel betreffen die erfindungsgemäße Verwendung und die erfindugsgemäße pharmazeutische Zusammensetzung entsprechend.

## Patentansprüche

1. Verwendung einer kosmetisch und dermatologisch verträglichen organischen Verbindung mit aktiviertem Sauerstoff zur Herstellung eines Präparates zur Verbesserung der Mikrozirkulation der Haut, wobei die Verbindungen mit aktiviertem Sauerstoff Acetalperoxide sind, die durch Ozonierung einer gesättigten oder ungesättigten Fettsäure oder eines Esters davon in Gegenwart eines Alkohols oder eines teilveretherten oder teilveresterten Polyols erhalten werden.

2. Verwendung nach Anspruch 1, wobei das Acetalperoxid durch Ozonierung von Sorbinsäure in Ethyldiglykol erhältlich ist.

3. Verwendung nach Anspruch 2, wobei das Acetalperoxid in Ethyldiglykol gelöst ist.

4. Verwendung nach einem der Ansprüche 1 bis 3, wobei das Acetalperoxid in einem Mittel enthalten ist in einer Menge von 0,5 bis 20 Gew.-%, bezogen auf das Gesamtgewicht des Mittels.

5. Pharmazeutische Zusammensetzung zur Verbesserung der Mikrozirkulation der Haut enthaltend Acetalperoxide gemäß einem der Ansprüche 1 bis 4 und eine Fettsäure und/oder einen Fettsäureester.

## Claims

1. Use of a cosmetically and dermatologically compatible organic compound with activated oxygen for the production of a preparation for improvement of the microcirculation of the skin, said compounds with activated oxygen being acetal peroxides which are obtained by ozonizing a saturated or unsaturated fatty acid or an ester thereof in the presence of an alcohol or a partly etherified or partly esterified polyol.

2. The use according to claim 1, said acetal peroxide being obtainable by ozonizing sorbic acid in ethyl diglycol.

3. The use according to claim 2, said acetal peroxide being dissolved in ethyl diglycol.

4. The use according to any of claims 1 to 3, said acetal peroxide being contained in an agent in an amount of 0.5 to 20 wt.-%, relative to the agent's overall weight.

5. A pharmaceutical composition for improvement of the microcirculation of the skin, containing acetal peroxides according to any of claims 1 to 4 and a fatty acid and/or a fatty acid ester.

## Revendications

1. Utilisation d'un composé organique bien toléré sur le plan cosmétique et dermatologique, comprenant de l'oxygène activé, pour fabriquer un produit destiné à améliorer la microcirculation de la peau, les composés avec l'oxygène activé étant des peroxydes acétals obtenus par ozonisation d'un acide gras saturé ou non saturé ou d'un ester de celui-ci en présence d'un alcool ou d'un polyol partiellement éthérisé ou partiellement estérifié.

2. Utilisation selon la revendication 1,
le peroxyde acétal pouvant être obtenu par ozonisation d'acide sorbique dans du diglycol éthylique.

3. Utilisation selon la revendication 2,
le peroxyde acétal étant dissout dans du diglycol éthylique.

4. Utilisation selon l'une quelconque des revendications 1 à 3,
le peroxyde acétal étant contenu dans un produit dans une quantité de 0,5 à 20 % en poids par rapport au poids total du produit.

5. Composition pharmaceutique pour améliorer la microcirculation de la peau, contenant des peroxydes acétals selon l'une quelconque des revendications 1 à 4 et un acide gras et/ ou un ester d'acide gras.
